# EUROPÄISCHE PATENTSCHRIFT

(11) **EP 1 096 947 B1**
(45) Veröffentlichungstag und Bekanntmachung des Hinweises auf die Patenterteilung: **04.08.2004**
(21) Anmeldenummer: 99925030.1
(22) Anmeldetag: 22.05.1999
(51) Int. Cl.: A61P 31/12, A61P 31/22, A61K 45/06, A61K 31/164, A61K 38/08, A61K 38/04

(54) **TOPISCH ANWENDBARES ARZNEIMITTEL ZUR BEHANDLUNG VON VIRUSINFEKTIONEN**
TOPICAL DRUG FOR THE TREATMENT OF VIRAL INFECTIONS
MEDICAMENT TOPIQUE POUR LE TRAITEMENT D'INFECTIONS VIRALES

(30) Priorität: 26.05.1998 DE 19823319
(43) Veröffentlichungstag der Anmeldung: 09.05.2001
(73) Patentinhaber: Engelhard Arzneimittel GmbH & Co. KG, 61138 Niederdorfelden (DE)
(72) Erfinder: RUNKEL, Frank, D-35418 Buseck (DE)
(74) Vertreter: Weller, Wolfgang, Dr.
(86) Internationale Anmeldenummer: PCT/EP1999/003540
(87) Internationale Veröffentlichungsnummer: WO 1999/061045

(56) Entgegenhaltungen:
- EP-A- 0 154 344
- EP-A- 0 355 536
- EP-A- 0 439 042
- WO-A-95/17165
- WO-A-96/29056
- DE-A- 2 429 033
- US-A- 5 624 906
- VUCINA ET AL: "Uso del Pantenol (Bepantol M.R.) en el Tratamiento del Herpes Zoster" PEDIATR A, Bd. 8, Nr. 2, 1965, Seiten 147-150, XP002120615
- HARTMANN D ET AL: "[Experimental protection with tyrothrizin against herpes simple virus infections in mice]. Schutzversuche mit Tyrothricin an der Herpes -simplex-Virus-infizierten Maus." ARZNEIMITTEL-FORSCHUNG, (1979) 29 (1) 50-4. , XP002120616

## Beschreibung

Die Erfindung betrifft ein topisch anwendbares Arzneimittel zur Behandlung von Virusinfektionen enthaltend einen die Vermehrung von Viren in einer Wirtszelle inhibierenden ersten Wirkstoff.

Viren sind Krankheitserreger, die sich nicht selbständig vermehren können, sondern zum Zwecke ihrer Vermehrung in lebende Zellen, sogenannte Wirtszellen, eindringen. Dort programmieren sie den zellulären Stoffwechsel derart um, daß eine Vielzahl von Nachkommen-Viren gebildet werden. Diese verlassen die Wirtszelle und befallen umliegende weitere Wirtszellen, wo sich der Vorgang wiederholt, und so fort.

Viren sind sehr einfach aufgebaute Krankheitserreger. Sie enthalten eine Nukleinsäure, die die genetische Information des Virus beinhaltet, und zwar entweder eine einzelsträngige oder eine doppelsträngige DNA oder RNA. Die Nukleinsäure ist von einem Proteinmantel, dem Capsid, umgeben. Der Komplex aus Nukleinsäure und Protein wird als Nukleocapsid bezeichnet. Bei den behüllten Viren ist das Nukleocapsid von einer Lipidmembran eingeschlossen.

Mit einer Länge von 20 bis 300 Nanometern sind Viren wesentlich kleiner als Bakterien und können daher bspw. aus Blut nicht über Bakterienfilter abgetrennt werden.

Die Klassifikation von Viren erfolgt einerseits nach ihrer Nukleinsäure in DNA- und RNA-Viren und andererseits nach dem Vorhandensein einer Lipidhülle in behüllte und unbehüllte Viren.

Eine Virusinfektion verläuft in mehreren definierten Phasen (siehe Pschyrembel, 258. Auflage, S. 1667):
1. Adsorption:
   In der Adsorptionsphase lagert sich das Virus an seine Wirtszelle an. Bei behüllten Viren, die in ihrer Lipidhülle meist viruseigene (Glyco-)Proteine tragen, erfolgt die Adsorption über eine spezifische Interaktion der Virushüllproteine mit Membranproteinen der Wirtszelle, den Virusrezeptoren. Diese Interaktion von Virushüllproteinen und zellulären Virusrezeptoren bestimmt das Wirtsspektrum des Virus. Viren können nämlich immer nur ganz bestimmte Zelltypen befallen, die die jeweiligen Virusrezeptoren tragen,.
2. Penetration und Uncoating:
   Zur Penetration, dem Eindringen des Virus in die Wirtszelle, verschmilzt die Virushülle mit der Zellmembran (behüllte Viren). Bei unbehüllten Viren wird das Virus auf andere Weise, bspw. durch Pinozytose, ins Zellinnere eingeschleust. Im Zellinneren zerfällt das Nukleocapsid in Nukleinsäure und Proteine, dieser Vorgang wird als Uncoating bezeichnet. Das Uncoating findet entweder im Cytoplasma oder im Zellkern statt.
3. Replikation:
   Bei der Replikation wird die viruseigene Nukleinsäure und, davon ausgehend, die viralen Proteine vielfach multipliziert. Die virale Replikation erfolgt nach den verschiedensten Mechanismen, wobei immer wirtszelleigene Enzyme, Nukleotide, Aminosäuren und Energie verwendet werden.
4. Maturation und Freisetzung:
   Die Nachkommen-Nukleinsäuren verbinden sich mit Virusproteinen zu neuen Nukleocapsiden (Virusreifung, Assembly). Dann erfolgt der Austritt der Nukleocapside aus der Wirtszelle nach unterschiedlichen Mechanismen. Behüllte Viren verlassen ihre Wirtszelle meist durch "Budding" (Knospung), wobei die Lipidhülle beim Austritt aus der Zelle aus der Zellmembran erworben wird. Bei diesem Vorgang werden auch die Virushüllproteine in die Lipidhülle eingelagert.

Die Nachkommen-Viren befallen dann Wirtszellen in der Nachbarschaft der ursprünglich befallenen Wirtszelle, wo sie sich wiederum vermehren und von dort aus weitere Zellen befallen.

Eine andere Form der Virusausbreitung besteht darin, daß die befallene Wirtszelle mit in der Nachbarschaft liegenden Wirtszellen verschmilzt und ein sogenanntes Syncycium bildet. Auf diese Weise erhalten die Viren neues Reservoir zur Vermehrung, ohne dabei die Zellen zu verlassen und im extrazellulären Raum dem Immunsystem des Wirtsorganismus ausgesetzt zu sein.

In der Regel geht die Wirtszelle aufgrund der Virusvermehrung, die die übrigen Synthesevorgänge der Zelle meist vollständig blockiert, zugrunde.

Bei den "Tumorviren" kann die Zelle auch derart umprogrammiert werden, daß eine ungehemmte Zellteilung eintritt. Damit sichert das Virus, daß eine große Anzahl von Wirtszellen, innerhalb derer es sich vermehren kann, bereitgestellt wird. Bei anderen, den "latenten" Viren, tritt nur eine sehr langsame oder zeitweise überhaupt keine Virusvermehrung ein. Ein latentes Virus kann jedoch durch verschiedene Stimuli zur Vermehrung reaktiviert werden.

Im Gegensatz zu der Vermehrung von Bakterien, die durch eine Vielzahl bekannter Antibiotika unterbunden werden kann, sind bisher nur wenige Mittel bekannt, die die Vermehrung von Viren verhindern.

Der Grund hierfür besteht darin, daß sich die Viren zu ihrer Vermehrung wirtseigene Mechanismen zunutze machen. Daraus folgt, daß die meisten Virostatika, also chemische Verbindungen, die zur Therapie von Virusinfektionen eingesetzt werden, auch für die Wirtszellen und somit den Wirtsorganismus toxisch sind.

Die bisher bekannten Virostatika beruhen auf drei Wirkprinzipien (siehe Pschyrembel, 258. Auflage, S. 1668):
a) Verhinderung der Viruspenetration und/oder des Uncoating (siehe oben Schritt 2). Dazu werden zyklische Amine wie bspw. Amantadin eingesetzt.
b) Verhinderung der Virusreplikation (siehe oben Schritt 3), z.B. durch Nukleosid-Analoga wie Aciclovir oder Zidovudin.
c) Verhinderung der Maturation und Freisetzung (siehe oben Schritt 4), bspw. mit Inhibitoren viruseigener Reifungsproteine. Inhibitoren der HIV-Protease sind hier als Beispiel zu nennen.

Zu den bekanntesten Virostatika zählt das Nucleosid-Analog Aciclovir, das die Vermehrung von Herpes simplex und verwandten Viren spezifisch blockiert (siehe Roche, Lexikon Medizin, Urban & Schwarzenberg, 3. Auflage, S. 13). Zur topischen Anwendung wird es bspw. als "Lippencreme" von einer Vielzahl verschiedener Anbieter verkauft.

Aus der EP-A-0 154 344 ist es bekannt, durch Viren der Herpes-Gruppe ausgelöste Infektionen mit Lidocain alleine zu behandeln oder die inhibierende Wirkung von Lidocain durch Panthenol oder Pantothensäure zu verstärken.

Es ist auch bekannt, Kombinationen verschiedener Virostatika zur Behandlung von HIV-Infektionen einzusetzen. Aufgrund der schweren Nebenwirkungen und dem Erfordernis einer systemischen Verabreichung, um die in Blutzellen replizierenden HI-Viren zu erreichen, sind derartige Kombinationstherapien jedoch nur bei lebensbedrohlichen Infektionen angezeigt.

Vor diesem Hintergrund ist es die Aufgabe der vorliegenden Erfindung, ein topisch anwendbares Arzneimittel zur Behandlung von Virusinfektionen bereitzustellen, mit dem eine effiziente Bekämpfung der Virusinfektion unter größtmöglicher Vermeidung von Nebenwirkungen erreicht werden kann.

Erfindungsgemäß wird diese Aufgabe bei einem Arzneimittel der eingangs genannten Art dadurch gelöst, daß in dem Arzneimittel Thyrothricin als ein die Vermehrung von Viren in einer Wirtszelle inhibierender erster Wirkstoff in Kombination mit Dexpanthenol als einen das Eindringen der Viren in eine weitere Wirtszelle inhibierenden zweiter Wirkstoff zur Herstellung eines topisch anwendbaren Arzneimittels zur Behandlung von Virusinfektionen verwendet wird.

Das erfindungsgemäße Arzneimittel besteht somit aus zumindest zwei Substanzen, die in zwei unterschiedliche Phasen der Virusvermehrung eingreifen, nämlich einerseits in die Phase des Eintretens des Virus in die Zelle (oben aufgeführte Phasen 1 und/oder 2), und andererseits in diejenigen Phasen der Virusvermehrung, die sich im Inneren der Zelle abspielen (oben aufgeführte Phasen 3 und/oder 4).

Der die Vermehrung der Viren in einer Wirtszelle inhibierende erst Wirkstoff kann bspw. in die intrazelluläre Replikation der Viren eingreifen, so daß die Produktion der viralen Nukleinsäuren und der viralen Proteine unterbunden wird.

Der zweite in dem erfindungsgemäßen Arzneimittel enthaltene Wirkstoff schützt die Zellen vor dem Eindringen der Viren und entzieht den Viren somit die Grundvoraussetzung ihrer Vermehrung. Da die Viren auf diese Weise gezwungen werden, außerhalb der Zelle zu verbleiben, haben sie keine Möglichkeit, sich vor dem wirtseigenem Immunsystem zu verstecken. Sie sind somit der wirtseigenden zellulären Immunantwort mit B- und/oder T-Lymphozyten ausgeliefert.

Mit dem erfindungsgemäßen Arzneimittel wird somit auch die körpereigene Abwehr gegen Virusinfektionen unterstützt und dafür gesorgt, daß das Immunsystem die Viren möglichst effizient eliminieren kann.

Unter einem Wirkstoff, der das Eindringen der Viren in die Wirtszelle inhibiert, nämlich Dexpanthenol wird auch ein solcher Wirkstoff verstanden, der das Verschmelzen einer virusinfizierten mit einer nicht infizierten Wirtszelle verhindert.

Da mit Hilfe von zwei Wirkstoffen unterschiedliche Phasen der Virusvermehrung blockiert werden, ist mit dem erfindungsgemäßen Arzneimittel eine wesentlich effizientere Bekämpfung einer Virusinfektion möglich, als es bisher denkbar war.

Das erfindungsgemäße Kombinationsarzneimittel kann auch dann zur Therapie von Virusinfektionen eingesetzt werden, wenn eine Anzahl von Wirtszellen bereits befallen ist und sich die Infektion bereits in einem fortgeschrittenen Stadium befindet. In diesem Stadium wird der Patient die Virusinfektion in der Regel erst bemerken. Die erfindungsgemäßen Wirkstoffe sorgen nun dafür, daß eine intrazelluläre Multiplikation der Viren ausbleibt und gleichzeitig der Eintritt der Viren in noch nicht infizierte Zellen verhindert wird. Eine Virusinfektion zeigt sich oft in Form von Hautveränderungen, bspw. Bläschen- oder Pustelbildung, Rötung etc., was mit starkem Juckreiz und Spannungsgefühl verbunden ist. Der von der Virusinfektion befallene Patient neigt dazu, die befallenen Stellen aufzukratzen und die Viren so über große Hautbereiche zu verteilen, wo die Viren neue Zellen befallen können. Die Anwendung des erfindungsgemäßen Arzneimittelzneimittels verhindert nun die weitere Ausbreitung der Viren. Darüber hinaus wird dem Immunsystem die Möglichkeit geboten, die außerhalb der Zellen befindlichen Viren in kurzer Zeit zu beseitigen.

Durch die topische Anwendung des erfindungsgemäßen Arzneimittels, also die lokale Anwendung des Mittels direkt an Ort und Stelle der Infektion und damit der aktiven Virusvermehrung und - ausbreitung, können die Nebenwirkungen auf ein Minimum reduziert werden.

Tyrothricin ist ein Gemisch aus zyklischen und linearen Polypeptiden, das zu 80% aus dem Antibiotikum Tyrocidin und zu 20% aus dem Antibiotikum Gramicidin, beide aus Bacillus brevis isoliert, besteht. Neben seiner antibakteriellen Wirkung ist auch eine antivirale Wirkung von Tyrothricin gegen Influenza-, Mumps und Herpes simplex-Viren bekannt.

Die Verwendung von Tyrothricin als die Vermehrung der Viren in der Wirtszelle inhibierendem Wirkstoff hat den Vorteil, daß Tyrothricin als topisch anwendbarer Wirkstoff bereits seit längerer Zeit eingesetzt wird und mit keinen schweren Nebenwirkungen verbunden ist.

Dexpanthenol ist der Alkohol der Pantothensäure, einer Vorstufe des in jeder menschlichen Zelle vorhandenen Energieträgers und Intermediats Coenzym A. Coenzym A spielt eine zentrale Rolle im Intermediär-Stoffwechsel von Säugerzellen und wird vor allem bei geschädigten Zellen, bspw. Hautzellen, in großen Mengen umgesetzt.

Coenzym A kann der Zelle nicht direkt zugeführt werden, da die Zellmembran für dieses Molekül nicht permeabel ist. Der Alkohol Dexpanthenol dringt hingegen leichter in die Zelle ein und wird dort zum Coenzym A aufgebaut.

In einer nachstehend gezeigten Studie hat sich nun überraschenderweise herausgestellt, daß Dexpanthenol ein die Zellwand schützendes Agens ist, das das Eindringen von Viren, insbesondere von behüllten DNA-Viren, behindert.

Dexpanthenol ist ein idealer Wirkstoff, da er physiologisch gut verträglich ist und im Körper intrazellulär zu Coenzym A aufgebaut wird, einem körpereigenen Stoff, wie oben erwähnt wurde.

Deshalb ist die Verwendung von Dexpanthenol nicht mit Nebenwirkungen verbunden.

Daß Dexpanthenol wie ein Schutzschild gegen die Infektion einer Zelle mit einem Virus wirken könnte, war vollkommen überraschend, da Dexpanthenol bisher lediglich zur Wundbehandlung und als Nahrungsmittelzusatz eingesetzt wurde.

Es versteht sich, daß das erfindungsgemäße topisch anwendbare Arzneimittel eine Reihe von Hilfsstoffen, wie bspw. Makrogol, Lactose, Vaseline, Paraffin oder ähnliches aufweisen kann.

In einer vorteilhaften Weiterbildung weist das Arzneimittel einen Gehalt an Cetylpyridiniumchlorid auf.

Cetlypyridiniumchlorid ist eine quarternäre Ammoniumbase, die die Oberflächenspannung vermindert und daher den Kontakt der Wirkstoffe des erfindungsgemäßen Arzneimittels mit den zu behandelnden Oberflächen verbessert. Besonders vorteilhaft ist dies, wenn das Arzneimittel im Bereich von Schleimhäuten eingesetzt wird. Cetylpyridiniumchlorid hat den weiteren Vorteil, desinfizierend zu wirken. Dadurch wird die antivirale Wirksamkeit des erfindungsgemäßen Arzneimittels zusätzlich gestärkt.

In einer weiteren vorteilhaften Ausgestaltung ist das erfindungsgemäße Arzneimittel als Salbe, als Gel, als Spray, als Puder oder als Tinktur formuliert.

Dazu weist das erfindungsgemäße Arzneimittel die bei diesen Formulierungen üblichen Hilfsstoffe auf.

Hierbei ist von Vorteil, daß diese Formulierungen besonders zur topischen, also lokalen Anwendung von Wirkstoffen geeignet sind.

Als Salbe, Gel oder Puder ist das erfindungsgemäße Arzneimittel vor allem im Hautbereich einsetzbar. Als Spray oder Tinktur kann es vorteilhaft im Bereich von Schleimhäuten, z.B. in der Mundhöhle oder im Genitalbereich eingesetzt werden.

Besonders bevorzugt wird das Arzneimittel zur Behandlung von Infektionen mit DNA-Viren, insbesondere mit behüllten DNA-Viren eingesetzt.

In einer vorteilhaften Weiterbildung wird das erfindungsgemäße Arzneimittel zur Therapie von Infektionen mit Viren der Familie der Herpesviridae eingesetzt.

In einer nachstehend vorgestellten Studie an Patienten hat sich nämlich herausgestellt, daß das erfindungsgemäße Kombinationspräparat besonders wirksam zur Therapie von Infektionen mit Mitgliedern der Herpesviridae einsetzbar ist. Die Familie der Herpesviridae umfaßt unter anderem das Varicella-Zoster-Virus, das Epstein-Barr-Virus sowie das Cytomegalovirus, die sämtlich vor allem auch bei Kindern eine breite Verbreitung haben.

Der berühmteste Vertreter der Herpesviridae ist das Herpes simplex-Virus, ein sehr weit verbreitetes Virus, das unangenehme und lebenslang immer wiederkehrende Bläschen im Mundbereich verursacht.

Das erfindungsgemäße Arzneimittel ist insbesondere dazu geeignet, gegen Infektionen mit Herpes simplex eingesetzt zu werden. Wie in der erwähnten Patientenstudie gezeigt werden konnte, weist das Kombinationspräparat eine hohe Wirksamkeit gegenüber Herpes simplex-Infektionen auf.

In einer weiteren vorteilhaften Ausgestaltung wird das erfindungsgemäße Arzneimittel zur Behandlung von Infektionen mit Viren der Familien der Poxviridae eingesetzt.

In die Familie der Poxviridae fällt u.a. das sehr verbreitete Molluscum contagiosum-Virus.

Dieses Virus ist ein noch nicht näher definiertes Mitglied der Familie der Poxviridae. Es ist weltweit verbreitet und vermehrt sich im Bereich von Hautzellen. Molluscum contagiosum-Infektionen treten häufig bei Kindern auf und führen gelegentlich zum Epidemien.

Eine Infektion mit dem Molluscum contagiosum-Virus äußert sich in derben, bis zu erbsgroßen Papeln mit zentraler Eindellung, den sogenannten Dellwarzen. Dellwarzen treten vor allem im Bereich des Gesichts und der Augenlider, am Hals, an den Achseln, sowie im seitlichen Thorax- und Genitalbereich auf.

Sehr häufig sind Dellwarzen auch mit der Hautkrankheit Neurodermitis assoziiert.

In einer nachstehend vorgestellten Studie an Patienten konnte nun festgestellt werden, daß das erfindungsgemäße Arzneimittel besonders wirksam bei der Behandlung von Infektionen mit Molluscum contagiosum-Viren und damit zur Behandlung von Dellwarzen einsetzbar ist.

Beim Aufplatzen von Dellwarzen werden nämlich Viren über einen großen Bereich der Umgebung der Dellwarze ausgestreut. Durch großflächiges Auftragen des erfindungsgemäßen Arzneimittels um den Bereich der Dellwarze herum kann die gesamte Umgebung vor einem Neubefall mit Viren geschützt werden. Dies ist auch dann möglich, wenn die Virusinfektion bereits fortgeschritten ist.

Auf diese Weise kann einer Neuinfektion über weite Bereiche der Haut vorgebeugt werden, so daß eine Neuentstehung von Dellwarzen sicher verhindert wird.

Zusätzlich wird die intrazelluläre Virusvermehrung selbst durch den ersten Wirkstoff des Kombinationsarzneimittels bekämpft.

Eine Ausbreitung des Molluscum contagiosum-Virus und der damit verbundenen Entstehung von Dellwarzen kann somit effizient und unter größtmöglicher Vermeidung von Nebenwirkungen bekämpft werden.

Auch zur Prophylaxe gegen die Entstehung von Dellwarzen, z.B. bei den stark gefährdeten Neurodermitikern, ist das erfindungsgemäße Arzneimittel geeignet.

Weitere Vorteile ergeben sich aus der nachstehenden Beschreibung.

Es versteht sich, daß die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Die Erfindung wird nun anhand der anliegenden Zeichnung und unter Bezugnahme auf die nachstehenden Beispiele näher erläutert. Es zeigen:
- Fig. 1: einen schematischen Überblick über den Verlauf einer Infektion von Wirtszellen mit behüllten Viren, in dem die Angriffspunkte der erfindungsgemäßen Wirkstoffe angezeigt sind;
- Fig. 2: ein Balkendiagramm, das das Ergebnis einer Studie über die Wirkung des erfindungsgemäßen Arzneimittels bei Herpes simplex (Beispiel 1) zeigt; und
- Fig. 3: ein Balkendiagramm, das das Ergebnis einer Studie über die Wirkung des erfindungsgemäßen Arzneimittels bei Molluscum contagiosum (Beispiel 2) zeigt.

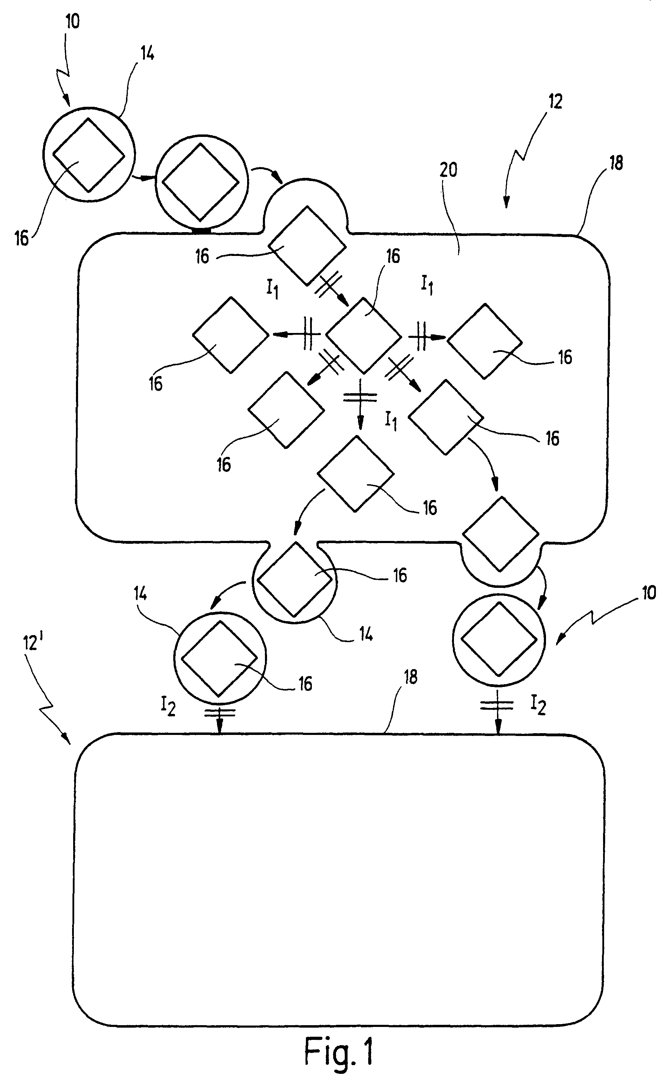

In Fig. 1 ist schematisch und in stark vereinfachter Weise ein Überblick über den Verlauf einer Infektion mit einem behüllten DNA-Virus, bspw. dem Herpes simplex-Virus, gezeigt.

Das Virus 10 nähert sich dabei zunächst einer ersten Wirtszelle 12, hier einer Hautzelle.

Das Virus 10 besteht aus einer Lipidhülle 14, die ein Nukleocapsid 16 umschließt. Das Nukleocapsid 16 weist auf nicht näher dargestellte Art und Weise die virale Nukleinsäure, die doppelsträngige Herpes simplex-DNA, sowie die viralen Nukleocapsid-Proteine auf.

Das Virus 10 dockt an der Wirtszelle 12 an, wobei Virushüllproteine mit zellulären Rezeptoren interagieren (nicht gezeigt). Dann dringt das Virus 10 in die Wirtszelle 12 ein, indem die Virushülle 14 mit der Zellmembran 18 der Wirtszelle 12 verschmilzt.

Das Nukleocapsid 16 wird in das Zellinnere 20 der Wirtszelle 12 entlassen. Im Zellinneren 20 erfolgt die Vermehrung des Virus 10. Dort wird eine große Menge an Nachkommen-Nukleocapsiden 16 produziert.

Die Nachkommen-Nukleocapside 16 treten aus der Wirtszelle 12 aus, indem sie die Zellmembran 18 ausstülpen und sich abschnüren (Budding). Die Virushülle 14 stammt somit von der Zellmembran 18 ab.

Die freigesetzten Nachkommen-Viren 10 können dann eine benachbarte weitere Wirtszelle 12' befallen.

Aus Fig. 1 ist ersichtlich, daß ein Wirkstoff oder Inhibitor I₁, der sich im Zellinneren 20 der befallenen Zelle 12 befindet, die intrazelluläre Vermehrung der Viren 10 inhibiert. Eine Neubildung von Nachkommen-Nukleocapsiden 16 wird somit durch I₁ verhindert.

Der zweite Wirkstoff oder Inhibitor I₂ verhindert dagegen das Eindringen von Viren 10 in weitere Wirtszellen 12'.

Auf diese Weise werden mit Hilfe der Wirkstoffe I₁ und I₂ zwei getrennte Schritte der Virusvermehrung inhibiert, und so erfolgt eine effizente Unterbindung der Vermehrung und Ausbreitung des Virus.

Die Wirksamkeit des erfindungsgemäßen Arzneimittels wird nachstehend anhand von zwei Beispielen belegt.

### Beispiel 1 Untersuchung der Wirkung einer Kombination aus Tyrothricin und Dexpanthenol bei Herpes simplex-Infektionen

Bei dieser Studie wurde anhand von 32 Patienten getestet, ob das erfindungsgemäße Kombinationspräparat mit einem Gehalt an Tyrothricin und Dexpanthenol bei Herpes simplex-Infektionen therapeutisch wirksam ist.

### Durchführung:

Die Patienten waren Kinder und Jugendliche im Alter von 7 bis 14 Jahren männlichen und weiblichen Geschlechts.

Dabei wurde eine Salbe angewendet, die je 1 g Salbe als Wirkstoffe 0,5 mg Tyrothricin, 25 mg Dexpanthenol und 0,25 mg Cetylpyridiniumchlorid enthielt.

Die Applikation der Salbe erfolgte im Durchschnitt zweimal täglich, die Medikation dauerte durchschnittlich 7 Tage. Behandelt wurden Herpes simplex-Infektionen im Lippenbereich, also die sogenannte Herpes labialis-Infektion.

Bei der Patientenauswahl wurde darauf geachtet, daß diese erstens keiner Behandlung mit weiteren Virostatika unterworfen waren, daß ihnen zweitens keine Corticosteroide verabreicht wurden und daß sie drittens nicht mit Immunsupressiva therapiert wurden.

Es wurde ferner darauf geachtet, daß die ersten Symptome der Herpes labialis-Infektion, nämlich ein Spannungsgefühl an den Lippen, vor maximal 12 Stunden auftrat.

Zur ärztlichen Beurteilung der Therapie wurden folgenden Faktoren bewertet:
1. Abschwellen der Bläschen
2. Größe von Juckreiz und Schmerzen
3. Austrocknungseffekt
4. Gewebsgranulation

Die Therapieergebnisse wurden anhand dieser Kriterien als sehr gut, gut, gering oder ungenügend eingestuft.

### Ergebnis:

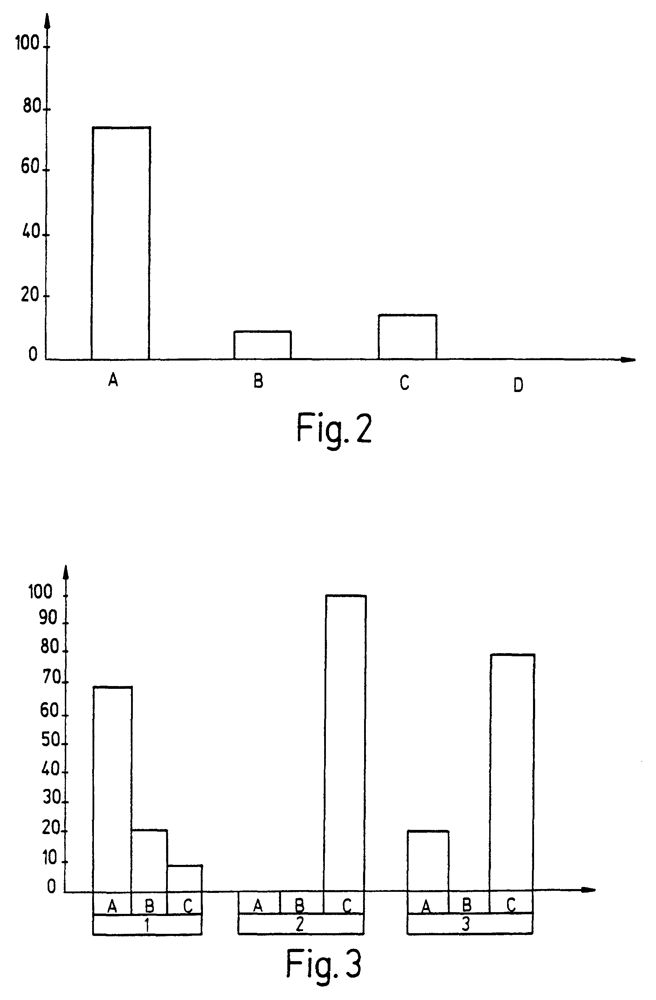

In der nachstehenden Tabelle 1 sind die Therapieergebnisse dieser Studie wiedergegeben. Die Ergebnisse sind in Fig. 2 in Form eines Balkendiagramms graphisch dargestellt.

Ein sehr guter Behandlungserfolg ist in Fig. 2 mit dem Buchstaben A gekennzeichnet, ein guter Behandlungserfolg mit dem Buchstaben B, ein geringer Behandlungserfolg mit dem Buchstaben C und ein ungenügender Behandlungserfolg mit dem Buchstaben D.

Da bei der Therapie spätestens nach dem 9. Tag ein Behandlungserfolg zu verzeichnen war, konnte bei keinem der Patienten der Erfolg als ungenügend eingestuft werden.

**Tabelle 1:**

| Ergebnisse der Therapie bei Herpes labialis unter Verwendung von Tyrothricin-Dexpanthenol-Salbe | | |
|---|---|---|
| Erfolg | Patientenzahl | Anteil am Gesamtkollektiv in% |
| sehr gut | 24 | 75 |
| gut | 3 | 10 |
| gering | 5 | 15 |
| ungenügend | 0 | 0 |

Insgesamt zeigt sich, daß ein Arzneimittel, das eine Kombination der Wirkstoffe Tyrothricin und Dexpanthenol enthielt, in 75 % der Fälle einen sehr guten Therapieerfolg bei der Behandlung von Herpes simplex-Infektionen im Lippenbereich erzielte. Bei weiteren 10 % der Patienten zeigte sich ein guter Behandlungserfolg.

Demnach hat das erfindungsgemäße Kombinationspräparat in 85 % der Fälle eine sehr gute oder gute Wirksamkeit bei der Bekämpfung von Herpes simplex-Infektionen.

### Beispiel 2: Untersuchung der Wirkung einer Kombination aus Tyrothricin und Dexpanthenol bei Molluscum contagiosum

In dieser Studie wurde eine Untersuchung zur Wirksamkeit des erfindungsgemäßen Arzneimittels mit den Wirkstoffen Tyrothricin und Dexpanthenol gegenüber einem weiteren Krankheitsbild, nämlich Molluscum contagiosum oder Dellwarzen, durchgeführt.

### Durchführung:

Die Untersuchungen wurden wie in Beispiel 1 durchgeführt, mit den folgenden Änderungen:

Das Patientenkollektiv umfaßte hier 40 Patienten. Davon wurden 20 mit der Salbe, die Tyrothricin (0,5 mg), Cetylpyrimidinium (0,25 mg) und Dexpanthenol (25 mg) enthielt, behandelt. Weitere 10 Patienten wurden mit einer Tyrothricin-Salbe (0,5 mg Tyrothricin, 0,25 mg Cetylpyridiniumchlorid, Tyrosur®-Salbe der Firma Engelhard) und weitere 10 Patienten mit Dexpanthenol-Salbe (25 mg Dexpanthenol, Bepanthen®-Salbe der Firma Hoffmann-LaRoche) behandelt.

Darüber hinaus wurde die Lokalisation der Dellwarzen am Patientenkörper festgehalten, wie in der nachstehenden Tabelle 2 gezeigt ist.

**Tabelle 2:**

| Lokalisation der Dellwarzen in Abhängigkeit des eingesetzten Arzneimittels | | | | | |
|---|---|---|---|---|---|
| Wirkstoff | Abdomen | Genital- und Anal- Bereich | Hals und Arme | Gesicht | Gesamtanzahl |
| Tyrothricin/Dexpanthenol | 5 | 3 | 1 | 11 | 20 |
| Dexpanthenol | 3 | 0 | 3 | 4 | 10 |
| Tyrothricin | 2 | 2 | 1 | 5 | 10 |

### Ergebnis:

Die Ergebnisse sind in der nachstehenden Tabelle 3 aufgeführt.

**Tabelle 3:**

| Therapeutische Wirksamkeit von Tyrothricin/Dexpanthenol bei Molluscum contagiosum im Vergleich zu Tyrothricin und Dexpanthenol alleine | | | | | | | | |
|---|---|---|---|---|---|---|---|---|
| Wirkstoff | Anzahl der Patienten | Anteil der Patienten in % | Erfolg | | | | | |
| | | | Abheilung | Abheilung (%) | Deutliche Besserung | Deutliche Besserung (%) | Geringe - ungenügende Besserung | Geringeungenügende Besserung (%) |
| Tyrothricin/ Dexpanthenol | 20 | 100 | 14 | 70 | 4 | 20 | 2 | 10 |
| Dexpanthenol | 10 | 100 | 0 | 0 | 0 | 0 | 10 | 100 |
| Tyrothricin | 10 | 100 | 2 | 20 | 0 | 0 | 8 | 80 |

Das in Tabelle 3 aufgeführte Ergebnis ist in Fig. 3 graphisch dargestellt.

Die unter 1 darstellten Säulen repräsentieren die Behandlungserfolge mit der Tyrothricin-Dexpanthenol-Salbe, unter 2 sind die Ergebnisse mit mit Dexpanthenol alleine und unter 3 die Ergebnisse Tyrothricin alleine aufgeführt.

Mit A ist die vollständige Abheilung der Dellwarzen bei den Patienten bezeichnet, B bezeichnet eine deutliche Besserung und C einen geringen bis ungenügenden Therapieerfolg.

Wie aus Tabelle 3 und Fig. 3 ersichtlich ist, zeigt das Kombinationspräparat aus Tyrothricin und Dexpanthenol in 70 % der Fälle eine vollständige Abheilung der Dellwarzen.

Eine deutliche Besserung war bei weiteren 20 % der Patienten nachweisbar, lediglich bei den restlichen 10 % der Patienten konnte nur ein geringer bis ungenügender Therapieerfolg festgestellt werden.

Im Vergleich dazu war bei Anwendung der Dexpanthenol-Salbe in 100 % der Fälle nur ein geringer bis ungenügender Therapieerfolg nachweisbar.

Bei Anwendung der Tyrothricin-Salbe wurde in 20 % der Fälle eine vollständige Abheilung der Dellwarzen erreicht.

Insgesamt ergibt sich, daß das Kombinationspräparat in 90 % der Fälle eine Abheilung oder deutliche Besserung der Dellwarzen bewirkt. In seiner Wirksamkeit gegenüber Molluscum contagiosum übertrifft das Kombinationspräparat somit bei weitem die wirkung der beiden Stoffe Tyrothricin und Dexpanthenol, wenn diese jeweils getrennt voneinander eingesetzt werden. Dies ist als ein unerwarteter synergistischer Effekt anzusehen.

## Patentansprüche

1. Verwendung von Tyrothricin als einen die Vermehrung von Viren in einer Wirtszelle (12) inhibierenden ersten Wirkstoff in Kombination mit Dexpanthenol als einen das Eindringen der Viren in eine weitere Wirtszelle (12') inhibierenden zweiten Wirkstoff zur Herstellung eines topisch anwendbaren Arzneimittels zur Behandlung von Virusinfektionen.

2. Verwendung nach Anspruch 1 zur Behandlung von Infektionen mit DNA-Viren, insbesondere mit behüllten DNA-Viren.

3. Verwendung nach Anspruch 2 zur Behandlung von Infektionen mit Viren (10) der Familie der Herpesviridae.

4. Verwendung nach Anspruch 3 zur Behandlung von Infektionen mit Herpes simplex.

5. Verwendung nach Anspruch 1 zur Behandlung von Infektionen mit Viren (10) der Familie der Poxviridae.

6. Verwendung nach Anspruch 5 zur Behandlung von Infektionen mit Molluscum contagiosum-Viren.

7. Verwendung nach Anspruch 6 zur Behandlung von Dellwarzen.

8. Verwendung nach einem der Ansprüche 1 bis 7, **dadurch gekennzeichnet, daß** zusätzlich ein Gehalt an Cetylpyridiumchlorid enthalten ist.

## Claims

1. Use of tyrothricin, as a first active compound which inhibits the multiplication of viruses in a host cell (12), in combination with Dexpanthenol, as a second active compound which inhibits the penetration of the viruses into an additional host cell (12'), for producing a topically utilizable pharmaceutical for treating virus infections.

2. Use according to Claim 1 for treating infections with DNA viruses, in particular with enveloped DNA viruses.

3. Use according to Claim 2 for treating infections with viruses (10) of the Herpesviridae family.

4. Use according to Claim 3 for treating infections with herpes simplex.

5. Use according to Claim 1 for treating infections with viruses (10) of the Poxviridae family.

6. Use according to Claim 5 for treating infections with molluscum contagiosum viruses.

7. Use according to Claim 6 for treating molluscum contagiosum.

8. Use according to one of Claims 1 to 7, **characterized in that** a content of cetylpyridium chloride is also present.

## Revendications

1. Utilisation de tyrothricine comme première substance active inhibant la multiplication des virus dans une cellule hôte (12) en combinaison avec du dexpanthénol en tant que deuxième substance active inhibant la pénétration des virus dans une autre cellule hôte (12') afin de produire un médicament à utilisation topique pour le traitement des infections virales.

2. Utilisation selon la revendication 1 pour le traitement d'infections par des virus à ADN, en particulier des virus à ADN enveloppés.

3. Utilisation selon la revendication 2 pour le traitement d'infections par des virus (10) de la famille des Herpes-virus.

4. Utilisation selon la revendication 3 pour le traitement d'infections par Herpes simplex.

5. Utilisation selon la revendication 1 pour le traitement d'infections par des virus (10) de la famille des Pox-virus.

6. Utilisation selon la revendication 5 pour le traitement d'infections par des virus de la famille de molluscum contagiosum.

7. Utilisation selon la revendication 6 pour le traitement des lésions cutanées dues à molluscum contagiosium.

8. Utilisation selon l'une des revendications 1 à 7, **caractérisée en ce qu'**une teneur en chlorure de cétylpyridium est ajoutée.
